# FASCICULE DE BREVET EUROPEEN

(11) **EP 0 381 548 B1**
(45) Date de publication et mention de la délivrance du brevet: **20.10.1993**
(21) Numéro de dépôt: 90400163.3
(22) Date de dépôt: 22.01.1990
(51) Int. Cl.: C07C 231/00, C07C 233/18, C11D 3/32

(54) **Procédé de préparation d'alkyldiéthanolamides gras purifiés, les produits obtenus et leurs utilisations**
Verfahren zur Herstellung von gereinigten, fetten Alkyldiethanolamiden, die daraus hergestellten Produkte und ihre Verwendung
Process for the preparation of pure, fatty alkyldiethanolamides, products thereof and their use

(30) Priorité: 31.01.1989 FR 8901219
(43) Date de publication de la demande: 08.08.1990
(73) Titulaire: S.E.P.P.I.C., SOCIETE D'EXPLOITATION DE PRODUITS POUR LES INDUSTRIES CHIMIQUES, F-75321 Paris Cédex 07 (FR)
(72) Inventeur: Boiteux, Jean-Pierre, F-81710 Saix (FR); Brancq, Bernard, F-78150 Le Chesnay (FR); Lecocu, Nelly, F-81100 Castres (FR); Loussayre, Frédéric, F-81710 Saix (FR)
(74) Mandataire: Portal, Gérard

(56) Documents cités:
- DE-B- 1 160 449
- GB-A- 972 440
- GB-A- 1 478 650
- US-A- 2 863 888
- US-A- 3 503 891
- PATENT ABSTRACTS OF JAPAN, vol. 2, no. 85, 12 juillet 1978; & JP-A-53 44513

## Description

Les alkyléthanolamides d'acides gras sont connus depuis des années et utilisés dans les formulations à usage cosmétique : shampooings, bains moussants..., à usage ménager : détergents liquides pour le lavage de la vaisselle à la main, ou à usage industriel.

Les alkyléthanolamides apportent à la formulation un effet épaississant intéressant sur le plan de la présentation du produit, favorisent le développement rapide de la mousse, accroissent la stabilité.

Les diéthanolamides d'acides gras, fabriqués selon les procédés classiques, contiennent toujours une certaine quantité de diéthanolamine libre, susceptible au cours du temps et en présence de dérivés nitrés de former des nitrosamines toxiques. De ce fait, les organisations européennes, comme le Colipa, ont recommandé, en particulier dans le domaine de la cosmétique et de l'hygiène, de limiter la teneur en diéthanolamine libre de ces produits à moins de 1 %.

Les alkyldiéthanolamides gras sont habituellement préparés par réaction des acides gras ou de leurs dérivés, esters méthyliques ou chlorures d'acides, sur la diéthanolamine.

Leur formule est : 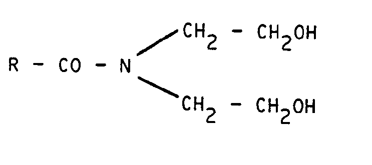
dans laquelle
R est un radical gras comprenant de 8 à 22 atomes de carbone.

Les amides les plus courants sont obtenus avec :
- les acides gras naturels de coprah ou de palmiste ;
- les acides gras de suif ou de palme ;
- les acides gras distillés sous forme de diverses coupes : C₁₂, C₁₂-C₁₄, C₁₂-C₁₆/C₁₆-C₁₈... ;
- les acides oléiques et oléo-cétyliques, ricinoléiques et undécyléniques ;
- les acides de synthèse ;
- et d'une façon générale, tous les acides gras naturels ou synthétiques habituellement utilisés dans l'industrie des agents de surface.

La synthèse des alkyldiéthanolamides, à partir d'acides gras ou de triglycérides, conduit à des produits dont la teneur en diéthanolamide libre est importante et, d'une façon générale, ces produits sont à présent préparés à partir des esters méthyliques des acides gras, ce qui permet de diminuer la teneur en diéthanolamine libre.

Dans le cas d'un alkyldiéthanolamide de coprah, le produit obtenu à partir d'esters méthyliques de coprah a les caractéristiques ci-dessous :
- teneur en amide : supérieure à 89 %
- diéthanolamine libre : 3 à 5 %
- pH en solution à 10 % : 9 à 11
- aspect : liquide trouble, hétérogène à température ordinaire.

Ce diéthanolamide de coprah est utilisé :
- comme agent stabilisateur de mousse dans les shampooings, bains moussants et diverses préparations nettoyantes ou détergentes contenant des produits tensioactifs anioniques ou amphotères ;
- pour augmenter la viscosité des préparations à base d'agents de surface anioniques et en particulier d'alkylsulfates.

La demanderesse a trouvé qu'un alkyldiéthanolamide gras, amélioré à la fois sur le plan de sa teneur en diéthanolamine, de son aspect et de son action irritante pour les muqueuses, pourrait être obtenu par acylation de l'amine libre.

Cette acylation peut être obtenue par emploi des dérivés acylants habituels : anhydrides ou chlorures d'acides, une préférence allant, compte tenu du coût et de la réactivité, à l'acétylation et à l'emploi de l'anhydride acétique comme agent acétylant.

Des essais d'acylation à l'aide :
. d'acétate d'éthyle
. d'esters méthyliques d'acides gras
. de diisocyanate d'hexaméthylène

ont conduit à une fixation insuffisante de l'amine libre ou à un produit qui, d'une stabilité insuffisante, laissait réapparaître au cours du vieillissement de la diéthanolamine libre.

Un diéthanolamide de coprah, préparé par réaction entre des esters méthyliques des acides gras totaux de coprah et la diéthanolamine, a une teneur en diéthanolamine libre comprise entre 3 et 5 %.

Cet amide, traité à l'aide d'anhydride acétique afin d'acétyler l'amine libre, puis soit soumis à un entraînement sous vide, soit à un lavage suivi d'un séchage pour éliminer l'acide acétique produit, conduit à un diéthanolamide de coprah dont la teneur en amine libre est inférieure à 1 % et le pH, en dispersion dans l'eau à 10 %, compris entre 6 et 8.

D'une façon surprenante, il a été constaté que ce diéthanolamide présentait des propriétés améliorées.

Sur le plan de la tolérance oculaire, mesurée sur oeil de lapin non rincé, selon le protocole français, il a été constaté une amélioration significative. L'indice est passé de 51,7 pour le diéthanolamide non traité à 21 pour le diéthanolamide traité à l'anhydride acétique.

Cette amélioration de la tolérance oculaire n'est pas liée à une alcalinité moindre du produit ; une solution de diéthanolamine à pH 10 a un test d'irritation oculaire de 2 et n'est donc pas irritante pour l'oeil et les essais d'irritation ayant été effectués au même pH.

Au point de vue présentation, le diéthanolamide de coprah après acétylation est un liquide limpide homogène à température ordinaire alors que le produit non acétylé contient une partie importante de produit concret donnant au produit un aspect hétérogène et trouble.

### Exemple 1

1 000 kg de diéthanolamide des acides gras totaux de coprah sont préparés en faisant réagir :
. 390 kg de diéthanolamine
. 720 kg d'esters méthyliques des acides gras totaux de coprah

en présence de 17 kg de méthylate de sodium en solution à 30 % dans de l'alcool méthylique.

L'ensemble étant maintenu à 80°C et sous vide.

La température est maintenue durant 5 à 6 heures et le méthanol, produit par la réaction, distillé en continu.

L'amide obtenu a une teneur en diéthanolamine libre d'environ 5 %. Cet amide est refroidi à 40-45°C, puis sous un vide de 300-400 torr (399,96.10² Pa - 533,28.10² Pa), 50 kg d'anhydride acétique sont introduits progressivement et sous agitation. L'introduction dure environ une heure. La réaction étant exothermique, la température est maintenue à 40-45°C à l'aide éventuellement d'un léger refroidissement.

Maintenir une heure sous vide à 40-45°C.

Vérifier que la teneur en amine libre est égale ou inférieure à 0,15 % sinon rajouter un peu d'anhydride acétique.

Mettre alors sous vide de 50 mm et porter à 90-92°C sous agitation. Lorsque l'amide a atteint cette température, mettre en route l'entraînement et le maintenir tant que l'indice d'acide est supérieur à 5.

L'amide obtenu est un liquide limpide ; la teneur en diéthanolamine libre est d'environ 0,5 %.

### Exemple 2

La même suite de réaction est appliquée à un diéthanolamide des acides gras étêtés de palmiste.

Les matières mises en oeuvre pour fabriquer 1 000 kg de cet amide sont :
. diéthanolamine : 340,9 kg
. esters méthyliques C₁₂-C₁₈ de palmiste : 750 kg
. méthylate de sodium à 30 % : 22,7 kg

La suite des opérations, étant menée exactement comme dans l'exemple 1, conduit à un amide se présentant sous la forme d'un liquide limpide et dont la teneur en amine libre est de l'ordre de 0,5 %.

Après chauffage à 40°C pendant deux semaines, la teneur en amine libre reste inchangée.

### Exemple 3

Un diéthanolamide oléique est préparé selon la même suite de réaction, les matières premières utilisées pour produire 1 000 kg de cet amide étant :
. diéthanolamine : 288 kg
. oléate de méthyle : 769 kg
. oléine : 8 kg
. méthylate de sodium à 30 % : 24 kg

L'amide obtenu a une teneur en amine libre inférieure à 1 %. Cette faible teneur est obtenue après lavage à l'eau et séchage sous vide du produit de la réaction.

## Revendications

1. Procédé de préparation de diéthanolamide d'acide gras à faible teneur en diéthanolamine, inférieure à 1 %, caractérisé en ce que l'on soumet à une réaction d'acylation, le produit brut obtenu par réaction connue entre la diéthanolamine et lesdits acides gras ou leurs dérivés ; et en ce que l'agent d'acylation utilisé est choisi parmi les anhydrides et les chlorures d'acides.

2. Procédé selon la revendication 1, caractérisé en ce que l'agent d'acylation utilisé est l'anhydride acétique.

3. Procédé selon la revendication 2, caractérisé en ce que la réaction d'acylation est réalisée à une température de 40 à 45°C sous une pression de 300 à 400 torr (399,96.10² - 533,28.10² Pa).

4. Procédé selon la revendication 1, caractérisé en ce que le produit brut précité est un diéthanolamide de coprah dont la teneur en diéthanolamine libre est comprise entre 3 et 5 %

5. Diéthanolamides d'acides gras à faible teneur en diéthanolamine, inférieure à 1%, susceptibles d'être obtenus par la mise en oeuvre du procédé selon l'une quelconque des revendications 1 à 4.

6. Utilisation des composés selon la revendication 5 dans des formulations contenant des agents moussants anioniques ou amphotères.

## Claims

1. Process for the preparation of fatty acid diethanolamides with a low diethanolamine content, less than 1%, characterized in that the raw product obtained by conventional reaction between the diethanolamine and said fatty acids or their derivatives, is subjected to an acylation reaction; and in that the acylating agent used is selected among the acid anhydrides and chlorides.

2. Process according to claim 1, characterized in that the acylating agent used is acetic anhydride.

3. Process according to claim 2, characterized in that the acylating reaction is carried out at a temperature of 40 to 45°C under a pressure of 300 to 400 torrs (399.96.10²-533.28 Pa).

4. Process according to claim 1, characterized in that said raw product is a diethanolamide of coprah of which the free diethanolamide content is comprised between 3 and 5%.

5. Fatty acid diethanolamides with a low diethanolamide content, less than 1%, which can be obtained with the process according to any one of claims 1 to 4.

6. Use of the products according to claim 5 in formulations containing anionic or amphoteric foaming agents.

## Patentansprüche

1. Verfahren zur Herstellung von Fettsäurediethanolamiden mit geringem Gehalt an Diethanolamin unter 1 %, dadurch gekennzeichnet,
daß das durch bekannte Umsetzung der entsprechenden Fettsäuren oder ihrer Derivate mit Diethanolamin erhaltene Rohprodukt acyliert und das verwendete Acylierungsmittel unter den Säureanhydriden und Säurechloriden ausgewählt wird.

2. Verfahren nach Anspruch 1, dadurch gekennzeichnet, daß als Acylierungsmittel Acetanhydrid verwendet wird.

3. Verfahren nach Anspruch 2, dadurch gekennzeichnet, daß die Acylierungsreaktion bei einer Temperatur von 40 bis 45 °C unter einem Druck von 300 bis 400 Torr (399,96·10² - 533,28·10² Pa) durchgeführt wird.

4. Verfahren nach Anspruch 1, dadurch gekennzeichnet, daß das eingesetzte Rohprodukt ein Kopra-Diethanolamid ist, dessen Gehalt an freiem Diethanolamin 3 bis 5 % beträgt.

5. Fettsäurediethanolamide mit geringem Gehalt an Diethanolamin unter 1 %, erhältlich nach dem Verfahren nach einem der Ansprüche 1 bis 4.

6. Verwendung der Verbindungen nach Anspruch 5 in Formulierungen, die anionische oder amphotere Schäummittel enthalten.
